# FASCICULE DE BREVET EUROPEEN

(11) **EP 4 106 726 B1**
(45) Date de publication et mention de la délivrance du brevet: **03.09.2025**
(21) Numéro de dépôt: 21715628.0
(22) Date de dépôt: 31.03.2021
(51) Int. Cl.: A61K 9/06, A61K 9/00, A61K 47/38, A61K 47/10

(54) **COMPOSITION PHARMACEUTIQUE TOPIQUE SOUS FORME DE GEL AQUEUX COMPRENANT AU MOINS DE L'AMITRIPTYLINE**
TOPISCHE PHARMAZEUTISCHE ZUSAMMENSETZUNG IN FORM EINES WÄSSRIGEN GELS MIT MINDESTENS AMITRIPTYLIN
TOPICAL PHARMACEUTICAL COMPOSITION IN THE FORM OF AQUEOUS GEL CONTAINING AT LEAST AMITRIPTYLINE

(30) Priorité: 06.04.2020 FR 2003425
(43) Date de publication de la demande: 28.12.2022
(73) Titulaire: Algotherapeutix, 92150 Suresnes (FR)
(72) Inventeur: PRINCIPE NICOLAS, Paola, 78720 Cernay La Ville (FR); LALLEMAND, Frédéric, 78120 Rambouillet (FR); THIROLOIX, Stéphane, 92150 Sursenes (FR)
(74) Mandataire: Casalonga
(86) Numéro de dépôt international: PCT/EP2021/058518
(87) Numéro de publication internationale: WO 2021/204634

(56) Documents cités:
- WO-A1-2016/057789
- FR-A1- 3 065 371
- US-A1- 2002 028 789
- DAVID J. KOPSKY ET AL: "High Doses of Topical Amitriptyline in Neuropathic Pain: Two Cases and Literature Review : Topical Amitriptyline in Neuropathic Pain", PAIN PRACTICE, vol. 12, no. 2, 16 June 2011 (2011-06-16), US, pages 148 - 153, XP055434409, ISSN: 1530-7085, DOI: 10.1111/j.1533-2500.2011.00477.x

## Description

La présente invention concerne une composition pharmaceutique topique sous forme de gel aqueux comprenant au moins de l'amitriptyline et/ou l'un de ses sels pharmaceutiquement acceptable, en une teneur comprise entre 10 et 30% en poids par rapport au poids total de la composition, de l'eau, au moins un polymère cellulosique et au moins un polyol en C₂-C₈.

La douleur neuropathique périphérique est causée par l'endommagement des structures nerveuses telles que les terminaisons nerveuses périphériques ou les nocicepteurs qui deviennent extrêmement sensibles à la stimulation et qui peuvent générer des impulsions en l'absence de stimulation.

Ces dommages peuvent être causés pour de nombreuses raisons comme les traumatismes, les maladies telles que le diabète, le zona et les cancers en phase avancée, les traitements chimio-thérapeutiques ou encore une brûlure chimique. La lésion du nerf périphérique peut conduire à des états pathologiques caractérisés par la présence de douleurs spontanées continues superficielles (sensation de brûlures ou de froid douloureux) ou profondes (sensation de compression ou d'étau), de douleurs paroxystiques (décharges électriques, coup de couteau) avec à l'examen clinique une hypoesthésie ou, au contraire une hyperalgésie (réponse accrue aux stimuli nocifs), une allodynie (douleur induite par un stimulus non douloureux) ou encore une hyperpathie (douleur persistante lors de stimulations répétées non nociceptive en temps normal). Les neuropathies peuvent également être associées à des signes sensitifs tels que les paresthésies, les engourdissements, le prurit.

Les neuropathies chimio induites sont particulièrement fréquentes, invalidantes et difficiles à traiter. Elles sont doses-dépendantes. Les atteintes des nerfs périphériques représentent la majorité des atteintes neurologiques liées à la toxicité des chimiothérapies. Elles sont la conséquence d'une atteinte toxique directe de l'axone ou d'une démyélinisation et représentent le facteur limitant le plus fréquent après la toxicité hématologique.

Ainsi, devant l'apparition de neuropathies chimio-induites, les doses de chimiothérapie seront réduites voire le traitement pourra être arrêté, constituant ainsi une réelle perte de chance pour le patient.

C'est ainsi que l'on a pu constater des neuropathies à la suite de traitement par des alcaloïdes (vincristine vinblastine, vinorelbine) entrainant souvent l'atteinte de petites fibres, les dérivés du platine (oxaliplatine, cisplatine, carboplatine), les anti-topoisomérase (VP16), les inhibiteurs du protéasome (bortézomib, carfilzomib), des dérivés de thalidomide comme le lénalidomide, les taxanes tels que le taxol ou le taxotère atteignant plutôt les grosses fibres. Il existe également des neuropathies après traitement par immunothérapie comme par exemple les anti-CD20, anti-CD30, anti-CD38.

Ces douleurs chimio induites opèrent selon des mécanismes mal connus, ainsi certains auteurs pensent qu'elles sont dues à une atteinte toxique directe sur l'axone sensoriel, à une démyélinisation ou encore à une altération du métabolisme calcique liées à l'atteinte des mitochondries, site d'action du paclitaxel et de la vincristine, par exemple.

Ainsi on sait que les taxanes interviennent au niveau du ganglion rachidien, des microtubules, des mitochondries et des terminaisons nerveuses, les sels de platines interviennent au niveau de la myéline et des canaux ioniques alors que les alcaloïdes interviennent au niveau de la myéline et des microtubules.

Ces douleurs neuropathiques sont souvent réfractaires aux traitements antalgiques usuels et entraînent des diminutions de doses voire des arrêts de chimiothérapie. Elles sont aujourd'hui prises en charge par des traitements per os comprenant les antidépresseurs (Amitriptyline, Duloxetine, Venlafaxine...) et/ou les antiépileptiques (Gabapentine, Pregabaline). Malheureusement, ces traitements systémiques induisent des effets secondaires majeurs (vertiges, somnolences, pertes de mémoire, sécheresse de la bouche voire rétention d'urine, nausées...) entraînant une mauvaise observance et un contrôle des douleurs peu satisfaisant.

Ces douleurs touchent en majorité les extrémités des mains et des pieds et entraînent une altération de la qualité de vie des patients considérable avec une impotence fonctionnelle pouvant aller jusqu'à l'impossibilité de marcher, des difficultés de préhension, un sommeil altéré, l'apparition d'un syndrome dépressif voire detendance suicidaire. Le retentissement sur la vie sociale et professionnelle peut être également très important.

L'intensité de la douleur est souvent qualifiée de sévère avec des patients qui évaluent leur douleur à plus de 7/10 sur l'Echelle Numérique Simple (douleur cotée de 0 à 10).

Les neuropathies post-zostériennes, sont d'origine différente et sont généralement liées à un endommagement des nerfs en raison d'une infection antérieure par le virus herpès zooster. Les nerfs endommagés ne sont plus capables detransmettre correctement les signaux de la peau vers le cerveau.

Les antidépresseurs tricycliques sont des composés chimiques découverts au début des années 1950. Ils sont largement utilisés pour traiter différents troubles psychiques en particulier la dépression, les troubles de panique, les troubles obsessionnels compulsifs, l'énurésie de l'enfant, les troubles bipolaires et l'hyperactivité. Ils sont également utilisés comme antalgiques.

Ces composés sont généralement administrés par voie orale.

L'amitriptyline est un antidépresseur tricyclique découvert en 1960 qui a été fréquemment recommandé comme traitement de première intention pour la dépression majeure, le syndrome de stress post-traumatique (TSPT), le trouble anxieux généralisé (TAG), la phobie sociale (PS), le trouble de panique, la fibromyalgie, les douleurs musculo-squelettiques chroniques, l'akinésie dans la maladie de Parkinson, la cataplexie, les migraines, la maladie de Parkinson, les symptômes vasomotrices de la ménopause, l'énurésie nocturne, le trouble dysphorique prémenstruel (TDPM), le trouble bipolaire, la boulimie, les troubles obsessionnels compulsifs (TOC) et les douleurs neuropathiques.

Par le passé, les patients étaient généralement traités par l'administration d'analgésiques pour soulager la douleur. La voie orale était alors largement privilégiée.

Cependant, l'administration par voie orale del'amitriptyline comme pour tous les antidépresseurs tricycliques présente de nombreux effets secondaires liés à leurs effets anti-cholinergique (risque d'hypotension artérielle, de tachycardie sinusale ou supra-ventriculaire, rarement BAV, d'une vision floue, d'une sécheresse buccale, de flush cutanés, de rétention aiguë d'urines ou de ralentissement du transit), anti-alpha adrénergique (risque de sédation, d'hypotension d'impuissance), inhibiteurs centraux des réflexes sympathiques ou encore stabilisant de membrane (effet pro-arythmogène) En particulier, un des effets redoutables et craint de l'amitriptyline est l'allongement du QT pouvant entraîner le décès d'un patient qui n'aurait pas été correctement surveillé.

En particulier, lors d'une administration par voie orale de l'amitriptyline pour le traitement des douleurs neuropathiques diabétiques, il a été rapporté des cas de sédation, d'hypotension orthostatique et des effets anti-cholinergiques ((cf. notamment Kiani et al, Iran J Pharm. Res. 2015 Fall ; 14(4) :1263-8). A long terme, les patients rapportent des troubles de la mémoire, des difficultés de concentration avec un important retentissement sur la qualité de leur travail ou sur leur vie quotidienne.

Par ailleurs, l'efficacité de l'amitriptyline par voie orale est lente (il faut 5 à 7 jours de traitement pour pouvoir commencer à apprécier l'efficacité du produit), variable en fonction des patients et incomplète. Il est par conséquent souvent nécessaire d'utiliser des combinaisons d'antalgiques pour pallier à ces inconvénients.

En outre, la prise orale d'antidépresseurs tricycliques a souvent mauvaise réputation auprès des patients du fait de leurs utilisations dans différents troubles psychiques.

Compte tenu des problèmes des traitements par voie orale, il a été tenté des traitements par voie topique. L'efficacité de l'amitriptyline par voie topique pour les douleurs neuropathiques n'a pas été démontrée. En particulier, l'article de Thomson et al « Systematic review of topical amitriptyline for the treatment of neuropathic pain », J. Clin. Pharm. Therm. 2015, 40, 496-503, conclut que des essais cliniques contrôlés révèlent que l'amitriptyline par voie topique n'est pas efficace dans le traitement de douleurs neuropathiques. La dose maximum utilisée est de 5% pour un malade atteint de sclérose en plaques et présentant des douleurs neuropathiques Également, l'article « A phase III randomized, placebo-controlled study of topical amitriptyline and ketamine for chemotherapy-induced peripherical neuropathy », Support Care Cancer, 2014 July ; 22(7) :1807-1814, a conclu qu'une composition topique comprenant 2 % en poids de kétamine et 4 % en poids d'amitriptyline n'était pas efficace pour traiter les douleurs neuropathiques post-chimiothérapie.

Aussi, la demande US 2002/028789 divulgue des compositions analgésiques sous forme de crème comprenant de l'amitriptyline et de la kétamine. La demande WO 2016/057789 divulgue des compositions pharmaceutiques à base d'α-amylase et pouvant éventuellement comprendre de l'amitriptyline, pour le traitement topique du prurit chez l'homme et l'animal. L'article scientifique de Kopsky et al, « High doses of topical amitriptyline in neuropathic pain : two cases and literature review : topical amitriptyline in neuropathic pain », vol 12, (2), 2011, pp 148-153 relate une étude clinique sur l'efficacité d'une application topique d'amitriptyline sous forme de crème pour le traitement des douleurs neuropathiques périphériques post-chimiothérapie.

Ainsi, il n'existe pas de traitement satisfaisant des douleurs neuropathiques, en particulier induites par la chimiothérapie. De plus des traitements combinant la kétamine et l'amitriptyline qui semblaient donner des résultats chez des patients présentant des douleurs neuropathiques post-zostérienne ou d'origine diabétique, n'ont pas permis de remédier aux douleurs neuropathiques induites par chimiothérapie, comme relevé dans l'étude clinique en phase III précitée.

Par ailleurs les doses envisagées, malgré le caractère invalidant de ces douleurs n'ont jamais dépassé 5 % que ce soit par voie orale ou topique.

De plus, les patients souffrant de neuropathies au niveau des extrémités (pieds et mains) présentent souvent une peau endommagée voire gercée et desséchée.

Parallèlement, la demande internationale WO 2018/197307 (*revendiquant la priorité de la demande de* brevet français FR3065371*)* précédemment déposée par Algotherapeutix, ainsi que l'article « Rossignol, J. et al. High concentration of topical amitriptyline for treating chemotherapy-induced neuropathies. Support Care Cancer 27, 3053-3059 (2019) » ont démontré l'efficacité d'une composition pharmaceutique sous forme de crème comprenant de 10% à 30% en poids d'amitriptyline pour son utilisation topique dans le traitement de douleurs neuropathiques périphériques. Les crèmes sont les formes galéniques couramment utilisées pour l'administration de principes actifs par voie topique car celles-ci procurent généralement un meilleur passage transdermique et une bonne solubilisation de tous les actifs. Toutefois, la stabilité physico-chimique de la composition selon la demande WO 2018/197307 sous forme de crème, et plus précisément sous forme d'émulsion huile-dans-eau, n'est pas satisfaisante, notamment pour son utilisation en tant que médicament.

Le chlorhydrate d'amitriptyline est une molécule amphiphile qui est soluble dans l'eau sous forme de sel. Or il a été découvert de façon surprenante que la présence de ces électrolytes est déstabilisant pour les émulsions huile-dans-eau, par masquage des charges de surface des globules d'huile ainsi que par rupture des équilibres d'interface huile/eau. Cette déstabilisation induit un déphasage de l'émulsion et à terme une séparation totale de l'huile et de l'eau. Dans le même temps, une réaction chimique se produit, se traduisant par un jaunissement de la couleur initialement blanche de l'émulsion. Ce déphasage et ce jaunissement sont problématiques pour une éventuelle mise sur le marché de la composition, notamment en tant que médicament.

Il existe donc un réel besoin de mettre à disposition une composition à base d'amitriptyline stable dans le temps, et efficace en application cutanée dans le traitement de la douleur, notamment dans le traitement des neuropathies périphériques et en particulier de neuropathies induites par chimiothérapie.

Il a été découvert de manière surprenante qu'une composition pharmaceutique sous forme de gel aqueux pour une application topique comprenant (i) au moins de l'amitriptyline et/ou l'un de ses sels pharmaceutiquement acceptable, dont la teneur totale en amitriptyline et/ou en l'un de ses sels pharmaceutiquement acceptable est comprise entre 10% et 30% en poids par rapport au poids total de la composition, (ii) de l'eau, (iii) au moins un polymère cellulosique, et (iv) au moins un polyol en C₂-C₈, et ayant un pH compris entre 4 et 7, était particulièrement stable dans le temps et permettait de traiter efficacement les douleurs, en particulier les douleurs neuropathiques périphériques post-chimiothérapie (ou CIPN pour « chemotherapy-induced peripheral neuropathy »), les douleurs neuropathiques post-zostériennes (ou PHN pour « post herpetic neuralgia ») ou encore les douleurs neuropathiques diabétiques (ou DPN pour « diabetic peripheral neuropathy »).

L'invention a donc pour objet une composition pharmaceutique sous forme de gel aqueux pour une application topique comprenant (i) au moins de l'amitriptyline et/ou l'un de ses sels pharmaceutiquement acceptable, dont la teneur totale en amitriptyline et/ou en l'un de ses sels pharmaceutiquement acceptable est comprise entre 10% et 30% en poids par rapport au poids total de la composition, (ii)de l'eau, (iii) au moins un polymère cellulosique, et (iv) au moins un polyol en C₂-C₈, et ayant un pH compris entre 4 et 7.

En particulier, la composition selon l'invention présente une bonne stabilité dans le temps à température ambiante (25°C) mais également à des températures de stockage plus élevées (45°C par exemple).

Il a également été constaté que la composition pharmaceutique sous forme de gel aqueux selon l'invention permet de faciliter la pénétration de l'amitriptyline à travers la peau, et ainsi d'obtenir une bonne efficacité thérapeutique.

La composition selon l'invention présente par ailleurs une biodisponibilité améliorée, de préférence à des concentrations en amitriptyline et/ou en l'un de ses sels pharmaceutiquement acceptables de 10% à 25% en poids et en particulier entre 10% et 20% en poids par rapport au poids total de la composition. En effet, à forte concentration l'amitriptyline a tendance à se réarranger, entrainant la formation d'agrégats susceptibles de limiter la biodisponibilité.

En outre, la composition selon l'invention comprend peu d'excipients, ce qui favorise une bonne tolérance locale de la composition (moins de risque d'allergie, moins de risque d'irritation).

La composition selon l'invention présente également de bonnes propriétés d'usage, à savoir la composition est translucide, inodore et agréable au toucher. En particulier, la composition selon l'invention présente un toucher non-gras comparativement aux émulsions divulguées dans la demande WO2018/197307.

De plus, la composition selon l'invention s'administre très facilement en flacon pompe contrairement aux émulsions huile-dans-eau. Ces flacons pompes sont notamment utiles pour assurer une bonne reproductibilité et une bonne précision de la dose administrée en principe actif.

Il a également été découvert de façon tout particulièrement surprenante que la composition pharmaceutique sous forme de gel aqueux pour une application topique selon l'invention, permettait de traiter efficacement l'érythromélalgie.

L'érythromélalgie est un acrosyndrome épisodique peu fréquent affectant principalement les deux membres inférieurs de manière symétrique par la présence d'érythème, de chaleur et de douleur brûlante. De nombreux articles scientifiques décrivent cette maladie orpheline, notamment « Leroux MB. Erythromelalgia: a cutaneous manifestation of neuropathy? An Bras Dermatol. 2018; 93(1) : 86-94 ».

L'application topique (par voie cutanée) de la composition selon l'invention résulte en un traitement efficace de l'érythromélalgie et des douleurs neuropathiques, plus particulièrement des douleurs neuropathiques périphériques tels que les douleurs neuropathiques périphériques post-chimiothérapie, post-zostériennes, et diabétiques.

Il a été constaté qu'une composition à base d'amitriptyline permet en plus de pallier aux douleurs, de retrouver une peau plus saine.

De plus, l'application topique de la composition selon l'invention présente peu, voire ne présente pas d'effets secondaires. En particulier, il n'est pas observé d'irritations cutanées à l'endroit d'application de la composition.

L'invention a également pour objet la composition selon l'invention pour son utilisation en tant que médicament, et plus particulièrement pour son utilisation par voie topique dans le traitement des douleurs neuropathiques tels que les douleurs neuropathiques périphériques.

D'autres objets, caractéristiques, aspects et avantages de l'invention apparaîtront encore plus clairement à la lecture de la description et de l'exemple qui suit.

Dans la présente description, et à moins d'une indication contraire :
- l'expression "au moins un" est équivalente à l'expression "un ou plusieurs" et peut y être substituée ;
- l'expression "compris entre...et..." est équivalente à l'expression "allant de... à..." et peut y être substituée, et sous-entend que les bornes sont incluses ;
- l'expression « polyoxyalkyléné » correspond, au sens de l'invention, à un motif -(O-alkyle)ₙ -, où n est un nombre entier variant de 2 à 200, de préférence de 2 à 40, plus préférentiellement de 2 à 20 ;
- l'expression « polyoxyéthyléné » correspond, au sens de l'invention, à un motif -(O-CH₂CH₂)ₙ-, où n est un nombre entier variant de 2 à 200, de préférence de 2 à 40, plus préférentiellement de 2 à 20.

La composition pharmaceutique selon l'invention est sous forme de gel aqueux.

Selon le Clinical Data Interchange Standards Consortium (CDISC), un gel pharmaceutique est une forme galénique semi-solide contenant un agent gélifiant pour donner de la rigidité à une solution ou à une dispersion colloïdale. Un gel peut contenir des particules en suspension.

Au sens de l'invention, il est entendu que les compositions sous forme de gel selon l'invention comprennent les compositions aqueuses visqueuses dont la viscosité est comprise entre 400 et 2500 mPa.s (à une température de 20°C et à pression atmosphérique).

De préférence, la viscosité des compositions sous forme de gel aqueux selon l'invention, à une température de 20°C et à pression atmosphérique, est comprise entre 400 et 2500 mPa.s ; plus préférentiellement entre 900 et 2000 mPa.s ; et plus préférentiellement encore entre 1000 et 1500 mPa.s.

A titre d'exemple, la viscosité des compositions sous forme de gel aqueux selon l'invention est déterminée au moyen d'un viscosimètre Brookfield LV, en utilisant le mobile numéro 63, tournant à la vitesse de 50 tpm (tours par minute), à une température 20,0°C +/- 2,0°C) dans un récipient de 30 mL, de 40 mm de hauteur et 35 mm de diamètre. Lorsque le viscosimètre est étalonné, le mobile est immergé dans le gel jusqu'à ce qu'un centimètre du fond de la bouteille. La viscosité est prise lorsque la mesure est stable.

La composition selon l'invention n'est pas sous forme d'émulsion, telle que par exemple une émulsion huile-dans-eau ou une émulsion eau-dans-huile. Autrement dit, la composition selon l'invention ne comprend pas de phase huileuse.

La composition selon l'invention n'est donc pas sous forme de crème.

Avantageusement, la composition selon l'invention est exempte de corps gras. Au sens de l'invention, on entend par « corps gras », un composé organique insoluble dans l'eau à 25°C et à pression atmosphérique (760 mm de Hg, soit 1,013.10⁵ Pa), c'est-à-dire de solubilité dans l'eau inférieure à 5% et de préférence inférieure à 1%, encore plus préférentiellement inférieure à 0,1%. A titre d'exemples de corps gras, on peut citer les cires, les hydrocarbures, les alcools gras comprenant de 9 à 40 atomes de carbone, les esters gras comprenant de 9 à 40 atomes de carbone, les éthers gras comprenant de préférence de 9 à 40 atomes de carbone, les silicones et leurs mélanges.

### Amitriptyline

La composition selon la présente invention comprend au moins de l'amitriptyline et/ou l'un de ses sels pharmaceutiquement acceptable.

Selon l'invention, la teneur totale en amitriptyline et/ou en l'un de ses sels pharmaceutiquement acceptable est comprise entre 10% et 30% en poids par rapport au poids total de la composition.

L'amitriptyline est de formule (I) suivante :

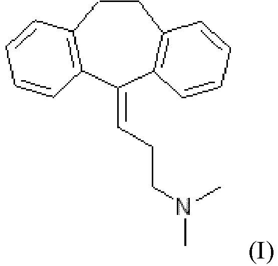

Dans le cadre de la présente invention, on entend par *« sels d'amitriptyline pharmaceutiquement acceptable »,* les sels compatibles avec une composition pharmaceutique c'est-à-dire destinée à être administrée à des humains. En particulier, on entend par sel d'amitriptyline pharmaceutiquement acceptable les hydrates, les solvates, les sels d'acides tel que les chlorhydrates et les clathrates de l'amitriptyline.

Comme sel tout particulièrement préféré de l'amitriptyline, on utilisera le chlorhydrate d'amitriptyline.

De préférence, la teneur totale en amitriptyline et/ou en l'un de ses sels pharmaceutiquement acceptable est comprise entre 10% et 25% en poids, plus préférentiellement entre 10% et 20% en poids, plus préférentiellement encore entre 10% et 15% en poids, par rapport au poids total de la composition.

Plus préférentiellement, la teneur totale en chlorhydrate d'amitriptyline est comprise entre 10% et 25% en poids, plus préférentiellement encore entre 10% et 20% en poids, encore mieux entre 10% et 15% en poids, par rapport au poids total de la composition.

Tout particulièrement, il a été observé de façon surprenante que lorsque la teneur totale en amitriptyline, et/ou en l'un de ses sels pharmaceutiquement acceptables tel que le chlorhydrate d'amitriptyline, est comprise entre 10% et 25% en poids, plus préférentiellement entre 10% et 20% en poids, par rapport au poids total de la composition selon l'invention, alors la biodisponibilité en amitriptyline de la composition selon l'invention est significativement améliorée.

En effet, il a été observé qu'à forte concentration l'amitriptyline a tendance à se réarranger, entrainant la formation d'agrégats susceptibles de limiter la biodisponibilité.

### Eau

La composition selon la présente invention comprend de l'eau.

De préférence, la teneur totale en eau est supérieure ou égale à 65% en poids, plus préférentiellement comprise entre 65 et 90% en poids; plus préférentiellement encore entre 70 et 90% en poids, encore mieux entre 75 et 85% en poids, par rapport au poids total de la composition.

### Les polymères cellulosiques

La composition selon l'invention comprend au moins un polymère cellulosique.

Par polymère « cellulosique », on entend selon l'invention tout composé polysaccharidique, substitué ou non, possédant dans sa structure des enchaînements de résidus glucose unis par des liaisons β-1,4 ; outre les celluloses non substituées, les dérivés de celluloses peuvent être anioniques, cationiques, amphotères ou non-ioniques.

Ainsi, les polymères cellulosiques utilisables selon l'invention peuvent être choisis parmi les celluloses non substituées y compris sous une forme microcristalline et les celluloses substituées.

Plus préférentiellement, les polymères cellulosiques utilisables selon l'invention ne comportent pas de chaîne grasse latérale en C₁₀-C₃₀ dans leur structure.

De préférence, le ou les polymères cellulosiques utilisables selon l'invention présentent un poids moléculaire moyen compris entre 5 000 et 1 500 000, plus préférentiellement entre 50 000 et 800 000, plus préférentiellement encore entre 400 000 et 800 000.

Parmi les polymères cellulosiques selon l'invention, on peut distinguer les éthers de celluloses, les esters de celluloses et les esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulfates d'éthylcellulose.

Parmi les éthers de cellulose non-ioniques, on peut citer les (C₁-C₄)alkylcelluloses telles que les méthylcelluloses et les éthylcelluloses (par exemple Ethocel standard 100 Premium de DOW CHEMICAL); les (poly)hydroxy(C₁-C₄)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes (poly)hydroxy(C₁-C₄)alkyl-(C₁-C₄)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Parmi les éthers de cellulose anioniques, on peut citer les (poly)carboxy(C₁-C₄)alkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les éthers de cellulose cationiques, on peut citer les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les (poly)hydroxy(C₁-C₄)alkyl celluloses, comme les hydroxyméthyl-, hydroxyéthyl- ou hydroxypropyl celluloses greffées notamment avec un sel de méthacryloyléthyl-triméthylammonium, de méthacrylmidopropyl-triméthylammonium, de diméthyl-diallylammonium. Les produits commercialisés répondant à cette définition sont plus particulièrement les produits vendus sous la dénomination « Celquat^{®} L 200 » et « Celquat^{®} H 100 » par la Société National Starch.

Selon un mode de réalisation préféré de l'invention, le ou les polymères cellulosiques sont choisis parmi les polymères cellulosiques ne comportent pas de chaîne grasse latérale en C₁₀-C₃₀ dans leur structure ; plus préférentiellement parmi les éthers de cellulose ; plus préférentiellement encore parmi les éthers de cellulose non-ioniques ; encore mieux parmi (a) les (C₁-C₄)alkylcelluloses telles que les méthylcelluloses et les éthylcelluloses, (b) les (poly)hydroxy(C₁-C₄)alkylcelluloses telles que les hydroxyméthylcelluloses, les hydroxyéthylcelluloses et les hydroxypropylcelluloses, (c) les celluloses mixtes (poly)hydroxy(C₁-C₄)alkyl-(C₁-C₄)alkylcelluloses telles que les hydroxypropyl-méthylcelluloses, les hydroxypropyl-éthylcelluloses, les hydroxyéthyl-méthylcelluloses, les hydroxyéthyl-éthylcelluloses et les hydroxybutyl-méthylcelluloses, et (d) leurs mélanges.

Plus préférentiellement, la composition selon l'invention comprend au moins une (poly)hydroxy(C₁-C₄)alkylcellulose telles que les hydroxyméthylcelluloses, les hydroxyéthylcelluloses et les hydroxypropylcelluloses ; encore mieux au moins l'hydroxyéthylcellulose.

De préférence, la teneur totale en polymère(s) cellulosique(s) est comprise entre 0,1 et 10% en poids, plus préférentiellement entre 0,5 et 5% en poids, plus préférentiellement encore entre 1 et 2,5% en poids, par rapport au poids total de la composition.

De préférence, la teneur totale en (poly)hydroxy(C₁-C₄)alkylcellulose(s) est comprise entre 0,1 et 10% en poids, plus préférentiellement entre 0,5 et 5% en poids, plus préférentiellement encore entre 1 et 2,5% en poids, par rapport au poids total de la composition.

De préférence, la teneur totale en hydroxyéthylcellulose est comprise entre 0,1 et 10% en poids, plus préférentiellement entre 0,5 et 5% en poids, plus préférentiellement encore entre 1 et 2,5% en poids, par rapport au poids total de la composition.

### Polyols

La composition selon la présente invention comprend au moins un polyol en C₂-C₈.

Par « polyol en C₂-C₈ » au sens de la présente invention, on entend un composé organique constitué d'une chaîne hydrocarbonée en C₂-C₈, éventuellement interrompue par un ou plusieurs atomes d'oxygène, et porteuse d'au moins deux groupements hydroxyles libres (-OH) portés par des atomes de carbone différents, ce composé pouvant être cyclique ou acyclique, linéaire ou ramifié, saturé ou insaturé, et à l'état liquide à température ambiante (25°C) et à pression atmosphérique (soit 1,013.10⁵ Pa).

De préférence, le ou les polyols en C₂-C₈ selon l'invention sont acycliques et non-aromatiques.

Les polyols en C₂-C₈ selon l'invention comprennent dans leur structure de 2 à 8 atomes de carbone, de préférence de 2 à 6 atomes de carbone, plus préférentiellement de 2 à 5 atomes de carbone.

Plus particulièrement, le ou les polyols utilisables selon l'invention comprennent de 2 à 10 groupements hydroxy, plus préférentiellement de 2 à 5 groupements hydroxy, plus préférentiellement encore de 2 à 3 groupements hydroxy.

De manière préférée, le ou lesdits polyols en C₂-C₈ utilisables selon l'invention sont choisis parmi les polyols en C₃-C₆, l'éthylène glycol, et leurs mélanges.

Selon un mode de réalisation préféré de l'invention, le ou lesdits polyols en C₂-C₈ utilisables selon l'invention sont choisis parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés ; plus préférentiellement la composition comprend au moins le propylène glycol.

De préférence, la teneur totale en polyol(s) en C₂-C₈ est comprise entre 0,1 et 15% en poids, plus préférentiellement entre 0,5 et 10% en poids, plus préférentiellement encore entre 1 et 6% en poids, et encore mieux entre 3 et 6% en poids, par rapport au poids total de la composition.

De préférence, la teneur totale en propylène glycol est comprise entre 0,1 et 15% en poids, plus préférentiellement entre 0,5 et 10% en poids, plus préférentiellement encore entre 1 et 6% en poids, et encore mieux entre 3 et 6% en poids, par rapport au poids total de la composition.

De préférence, le rapport pondéral de la teneur totale en polyol(s) en C₂-C₈ d'une part sur la teneur totale polymère(s) cellulosique(s) d'autre part, va de 0,01 à 150, plus préférentiellement de 0,1 à 20, plus préférentiellement encore de 0,4 à 6, mieux encore de 1 à 6, voire de 1,2 à 6.

Avantageusement, la teneur totale en poids en polymère(s) cellulosique(s) est strictement inférieure à la teneur totale en poids en polyol(s) en C₂-C₈.

### Les tensioactifs

La composition selon la présente invention peut éventuellement comprendre en outre au moins un tensioactif.

Les tensioactifs utilisables selon l'invention peuvent être choisis parmi les tensioactifs anioniques, les tensioactifs cationiques, les tensioactifs amphotères et/ou zwittérioniques, les tensioactifs non ioniques, et leurs mélanges.

Plus préférentiellement, le ou les tensioactifs utilisables selon l'invention sont choisi parmi les tensioactifs non ioniques.

Les tensioactifs non ioniques utilisables selon l'invention peuvent être choisis parmi les alkyl polyglucosides (APG), les esters de glycérol oxyalkylénés, les esters d'acides gras et de sorbitan oxyalkylénés, les esters d'acides gras polyoxyalkylénés (en particulier polyoxyéthylénés et/ou polyoxypropylénés) éventuellement en association avec un ester d'acide gras et de glycérol comme le mélange PEG-100 Stearate / Glyceryl Stearate commercialisé par exemple par la société ICI sous la dénomination Arlacel 165, les esters de sucre oxyalkylénés, et leurs mélanges.

Comme alkylpolyglucosides, on peut citer ceux contenant un groupe alkyle comportant de 6 à 30 atomes de carbone et de préférence de 8 à 16 atomes de carbone, et contenant un groupe glucoside comprenant de préférence 1,2 à 3 unités de glucoside. Les alkylpolyglucosides peuvent être choisis par exemple parmi le decylglucoside (Alkyl-C₉/C₁₁-polyglucoside (1.4)) comme le produit commercialisé sous la dénomination Mydol 10^{®} par la société Kao Chemicals ou le produit commercialisé sous la dénomination Plantacare 2000 UP^{®} par la société Cognis ; le caprylyl/capryl glucoside comme le produit commercialisé sous la dénomination Plantacare KE 3711^{®} par la société Cognis ; le laurylglucoside comme le produit commercialisé sous la dénomination Plantacare 1200 UP^{®} par la société Cognis ; le cocoglucoside comme le produit commercialisé sous la dénomination Plantacare 818 UP^{®} par la société Cognis ; le caprylylglucoside comme le produit commercialisé sous la dénomination Plantacare 810 UP^{®} par la société Cognis ; et leurs mélanges.

Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LI-S 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Gold schmidt ; les stéarates de glycéryle oxyéthylénés ; et leurs mélanges.

Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.

De préférence, le nombre de moles d'oxyde d'alkylène des tensioactifs non ioniques utilisables selon l'invention varie de 2 à 400 ; plus préférentiellement de 4 à 250.

De préférence, la composition selon l'invention est exempte de tensioactif.

Selon un mode de réalisation particulier de l'invention, la composition comprend au moins un tensioactif non ionique ; plus préférentiellement un tensioactif non ionique choisi parmi les esters de glycérol polyoxyalkylénés ; plus préférentiellement encore au moins un tensioactif non ionique choisi parmi les esters de glycéryle et d'acides gras hydrogénés et polyoxyéthylénés tel que le PEG-200 hydrogenated glyceryl palmate, les cocoates de glycéryle polyoxyéthylénés tels que le PEG-7 glyceryl cocoate et le PEG-30 glyceryl cocoate, les stéarates de glycéryle polyoxyéthylénés, et leurs mélanges.

Plus préférentiellement encore selon ce mode de réalisation, la composition selon l'invention comprend au moins un cocoate de glycéryle polyoxyéthyléné.

De préférence, lorsque la composition selon l'invention comprend au moins un tensioactif, la teneur totale en tensioactif(s) est comprise entre 0,1 et 10% en poids, plus préférentiellement entre 0,5 et 5% en poids, plus préférentiellement encore entre 1 et 4% en poids, par rapport au poids total de la composition.

De préférence, lorsque la composition selon l'invention comprend au moins un tensioactif, la teneur totale en tensioactif(s) non ionique(s) est comprise entre 0,1 et 10% en poids, plus préférentiellement entre 0,5 et 5% en poids, plus préférentiellement encore entre 1 et 4% en poids, par rapport au poids total de la composition.

De préférence, lorsque la composition selon l'invention comprend au moins un tensioactif, la teneur totale en ester(s) de glycérol (poly)oxyalkyléné(s) est comprise entre 0,1 et 10% en poids, plus préférentiellement entre 0,5 et 5% en poids, plus préférentiellement encore entre 1 et 4% en poids, par rapport au poids total de la composition.

Selon un mode de réalisation préféré de l'invention, la composition est exempte d'agent anti-oxydant.

Selon une variante del'invention, la composition comprend en outre au moins un agent anti-oxydant ; plus préférentiellement choisi parmi le tocophérol et ses esters, tels que l'acétate de tocophérol, le gallate de propyle, l'hydroxytoluène butylée (BHT), l'hydroxyanisole butylé (BHA), et leurs mélanges.

Selon un mode de réalisation préféré de l'invention, la composition est exempte d'agent séquestrant.

Selon une variante del'invention, la composition comprend en outre au moins un agent séquestrant ; plus préférentiellement choisi parmi (a) l'acide éthylènediamine tétracétique (EDTA) et ses sels tel que le sel disodique d'éthylène diaminetétracétique (Disodium EDTA), (b) les dérivés phosphoniques et leurs sels tels que l'acide hexaméthylène diaminetétra(méthylène phosphonique), l'acide éthylènediamine tétra(méthylène phosphonique), l'acide 1-hydroxyéthylidène-1,1-diphosphonique, l'acide aminotri(méthylènephosphonique), l'acide diéthylène-triamine penta(méthylène phosphonique), (c) les polymères polyaminés tels que les polyalkylène polyamines et leurs dérivés, en particulier la polyéthylèneimine, (d) les dendrimères à activité chélatante, (e) les protéines comme la spermine, la spermidine, la transférine, la ferritine, (f) les acides carboxyliques comme l'acide phytique, l'acide citrique, l'acide malique, l'acide nitrilo-acétique, l'acide fumarique, l'acide tartrique, l'acide succinique, l'acide oxalique, (g) la desferrioxamine mesylate, et leurs mélanges.

La définition d'« agent séquestrant » (également appelé « agent chélatant ») est bien connu de l'homme du métier et fait référence à un composé ou un mélange de composés capable(s) de former un chélate avec un ion métallique. Un chélate est un complexe inorganique dans lequel un composé (l'agent séquestrant ou chélatant) est coordiné à un ion métallique, c'est-à-dire qu'il forme une ou plusieurs liaisons avec l'ion métallique (formation d'un cycle incluant l'ion métallique).

Un agent séquestrant (ou chélatant) comprend généralement au moins deux atomes donneurs d'électrons qui permettent la formation de liaisons avec l'ion métallique.

Selon un autre mode de réalisation particulier de l'invention, la composition comprend au moins un agent séquestrant et au moins un agent anti-oxydant.

Selon une autre variante de l'invention, la composition est exempte de corps gras, d'agent séquestrant et/ou d'agent anti-oxydant.

Le pH de la composition selon l'invention est compris entre 4 et 7, plus préférentiellement entre 5 et 6.

Le pH de ces compositions peut être ajusté à la valeur désirée au moyen d'agents alcalinisants ou d'agents acidifiants habituellement utilisés. Parmi les agents alcalinisants, on peut citer, à titre d'exemples, l'ammoniaque, les alcanolamines, les hydroxydes minéraux ou organiques. Parmi les agents acidifiants, on peut citer, à titre d'exemples, les acides minéraux ou organiques comme l'acide chlorhydrique, l'acide orthophosphorique, les acides carboxyliques comme par exemple l'acide acétique, l'acide tartrique, l'acide citrique, l'acide lactique, les acides sulfoniques.

La composition selon l'invention peut contenir en outre des additifs habituellement utilisés en pharmaceutique, comme un ou plusieurs parfums, tampons, des colorants, des antibactériens et/ou antifongiques.

Comme antibactérien, les parabènes sont de préférence utilisés, et plus préférentiellement le méthyl-parabène.

Ces additifs peuvent être présents dans la composition selon l'invention en une quantité allant de 0 à 20 % en poids par rapport au poids total de la composition.

L'homme de métier veillera à choisir ces éventuels additifs et leurs quantités de manière à ce qu'ils ne nuisent pas aux propriétés des compositions de la présente invention.

Dans un mode de réalisation particulièrement préférée de l'invention, la composition pharmaceutique sous forme de gel aqueux pour une application topique comprend :
- de 10 à 30% en poids, de préférence de 10 à 25 % en poids, plus préférentiellement de 10 à 20% en poids, plus préférentiellement encore de 10 à 15% en poids, d'amitriptyline ou de l'un de ses sels pharmaceutiquement acceptable, par rapport au poids total de la composition,
- de 0,1 à 10% en poids, de préférence de 0,5 à 5% en poids, plus préférentiellement encore de 1 à 2,5% en poids, d'au moins un polymère cellulosique tel que décrit précédemment, par rapport au poids total de la composition,
- de 0,1 à 15% en poids, de préférence de 0,5 à 10% en poids, plus préférentiellement encore de 1 à 6% en poids, et encore mieux de 3 à 6% en poids, d'au moins un polyol en C₂-C₈ tel que décrit précédemment, par rapport au poids total de la composition,
- optionnellement de 0,1 à 10% en poids, plus préférentiellement de 0,5 à 5% en poids, plus préférentiellement encore de 1 à 4% en poids, d'au moins un tensioactif non ionique tel que décrit précédemment, par rapport au poids total de la composition,
- optionnellement de 0 à 3% en poids au moins un agent séquestrant et/ou au moins un agent anti-oxydant tels que décrits précédemment,
- de 0 à 1% en poids d'un ou plusieurs ajusteurs de pH tel que décrit précédemment, de façon à maintenir le pH entre 4 et 7, plus préférentiellement entre 5 et 6.
- de l'eau, en une teneur totale supérieure ou égale à 65% en poids, de préférence comprise entre 65 et 90% en poids ; plus préférentiellement entre 70 et 90% en poids, plus préférentiellement encore entre 75 et 85% en poids, par rapport au poids total de la composition.

Selon une variante de ce mode de réalisation, la composition est exempte de corps gras, de tensioactif, d'agent séquestrant et/ou d'agent anti-oxydant.

Dans un mode de réalisation tout particulièrement préférée de l'invention, la composition pharmaceutique sous forme de gel aqueux pour une application topique comprend :
- de 10 à 30% en poids, de préférence de 10 à 25 % en poids, plus préférentiellement de 10 à 20% en poids, plus préférentiellement encore de 10 à 15% en poids, d'amitriptyline ou de l'un de ses sels pharmaceutiquement acceptable, par rapport au poids total de la composition,
- de 0,1 à 10% en poids, de préférence de 0,5 à 5% en poids, plus préférentiellement encore de 1 à 2,5% en poids, d'au moins un éther de cellulose non-ionique, de préférence de poids moléculaire moyen compris entre 50 000 et 800 000, tel que décrit précédemment, par rapport au poids total de la composition,
- de 0,1 à 15% en poids, de préférence de 0,5 à 10% en poids, plus préférentiellement encore de 1 à 6% en poids, et encore mieux de 3 à 6% en poids, d'au moins un polyol en C₂-C₈ choisi parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés, par rapport au poids total de la composition,
- optionnellement de 0,1 à 10% en poids, plus préférentiellement de 0,5 à 5% en poids, plus préférentiellement encore de 1 à 4% en poids, d'au moins un ester de glycérol oxyalkyléné tel que décrit précédemment, par rapport au poids total de la composition,
- optionnellement de 0 à 3% en poids au moins un agent séquestrant et/ou au moins un agent anti-oxydant tels que décrits précédemment,
- de 0 à 1% en poids d'un ou plusieurs ajusteurs de pH tel que décrit précédemment, de façon à maintenir le pH entre 4 et 7, plus préférentiellement entre 5 et 6.
- de l'eau, en une teneur totale supérieure ou égale à 65% en poids, de préférence comprise entre 65 et 90% en poids ; plus préférentiellement entre 70 et 90% en poids, plus préférentiellement encore entre 75 et 85% en poids, par rapport au poids total de la composition.

Selon une variante de ce mode de réalisation, la composition est exempte de corps gras, de tensioactif, d'agent séquestrant et/ou d'agent anti-oxydant.

Les compositions selon ces modes de réalisation particulièrement préférés sont particulièrement efficaces dans le traitement de l'érythromélalgie et des douleurs neuropathiques périphériques, notamment les douleurs neuropathiques périphériques induites par chimiothérapie.

Ces compositions selon ce mode de réalisation sont particulièrement stables. Ces compositions ont été soumises à des études de stabilités en conditions de température ambiante (25°C) et accélérée (40°C) pendant 6 mois. Il résulte de ces études que ces compositions n'ont pas changé d'aspect visuel ni chimiquement (dosage du principe actif et des produits de dégradation).

Une composition préférée selon l'invention a également été soumise à des dégradations forcées dans des conditions acidité forte, alcalinité forte, chaleur, lumière et condition oxydative. Les produits de dégradations observés sont restés dans des seuils acceptables.

L'invention a également pour objet une composition selon l'invention telle que décrite précédemment pour son utilisation en tant que médicament.

L'invention a aussi pour objet une composition selon l'invention telle que décrite précédemment pour son utilisation par voie topique dans le traitement des douleurs neuropathiques ; de préférence pour son utilisation par voie topique dans le traitement des douleurs neuropathiques périphériques ; plus préférentiellement pour son utilisation par voie topique dans le traitement des douleurs neuropathiques périphériques post-chimiothérapie, des douleurs neuropathiques post-zostériennes, des douleurs neuropathiques diabétiques ; plus préférentiellement encore pour son utilisation par voie topique dans le traitement des douleurs neuropathiques périphériques post-chimiothérapie.

L'invention a également pour objet une composition selon l'invention telle que décrite précédemment pour son utilisation par voie topique dans le traitement de l'érythromélalgie.

L'invention porte également sur une composition selon l'invention telle que décrite précédemment pour son utilisation dans le traitement de cancers comportant des séances de chimiothérapie, la composition étant administrée par voie topique entre les séances de chimiothérapie pour remédier ou prévenir des douleurs neuropathiques, en particulier périphériques, susceptibles d'être induites par la chimiothérapie.

L'invention porte également sur une composition selon l'invention telle que décrite précédemment pour son utilisation pour remédier ou prévenir des douleurs neuropathiques, en particulier périphériques, susceptibles d'être induites par la chimiothérapie.

Les exemples suivants illustrent la composition selon l'invention et les avantages de cette composition. Cependant, ils ne représentent en rien une limitation de la présente invention mais illustrent simplement l'invention.

### Exemples :

### Exemple 1 :

Etude *ex vivo* comparée del'absorption percutanée de l'amitriptyline dans une formulation A sous forme de gel aqueux (invention) et dans une formulation B sous forme de crème (comparatif).

Le gel aqueux (composition A) suivant a été préparé à partir des ingrédients indiqués dans le tableau ci-après dont les quantités sont exprimées en % en poids.

**[Tableau 1]**

| **COMPOSITION A (Invention)** | **Quantité** |
|---|---|
| Chlorhydrate d'amitriptyline | 10 |
| Hydroxyéthylcellulose | 1 |
| Propylène glycol | 5 |
| PEG-7 Cocoate de glycéryle | 2 |
| Sel disodique d'éthylène diaminetétracétique | 0,1 |
| Gallate de propyle | 0,05 |
| Agent de pH | Qsp pH 5,5 ± 0,5 |
| Eau | Qsp 100 |

La composition B (crème) comprend 10% en poids de chlorhydrate d'amitriptyline et 90% en poids de crème Excipial Hydrocrème^{®}, commercialisée par la société Galderma, par rapport au poids total de la composition B.

Chacune des compositions A et B a été appliquées sur des échantillons distincts de peau humaine. Pour chaque composition, l'expérience a été répétée 3 fois avec 3 échantillons de peau de 3 donneurs différents, soit 9 échantillons.

Les échantillons de peau sont montés dans une cellule deFrantz et sont portées à une température de surface de 32°C±1°C.

La composition A ou B est étendue de façon homogène à l'aide d'une spatule sur chaque échantillon de peau à raison de 10 mg par cellule, correspondant à 5mg /cm² de peau.

On rince les échantillons de peau 16 heures après application.

Chaque échantillon de peau a été placé avec une pince à épiler sur un papier absorbant (derme vers le bas).

On enlève le stratum corneum en utilisant des bandes adhésives.

Après élimination du stratum corneum l'échantillon est perforé. L'épiderme est ensuite séparé du derme. Chacun d'eux est placé dans des fioles séparées.

On a procédé ensuite à l'extraction des différents échantillons.

Ce profil de pénétration a démontré son efficacité en clinique lors de l'étude décrite par l'article de Rossignol *et al* précédemment cité.

Les résultats de ces extractions sont regroupés dans le tableau ci-dessous.

**[Tableau 2]**

| | **Composition A (Invention)** | **Composition B (Comparatif)** |
|---|---|---|
| Concentration d'amitriptyline restant à la surface de la peau - stratum corneum *(µg)* | 2,4 ± 1,5 | 3,3 ± 1,6 |
| Concentration d'amitriptyline dans l'épiderme *(µg)* | 3,6 ± 2,6 | 4,1 ± 2,5 |
| Concentration d'amitriptyline dans le derme *(µg)* | 5,2 ± 2,5 | 4,4 ± 2,2 |
| Concentration d'amitriptyline dans le fluide récepteur (circulation sanguine) | 0,15 ± 0,08 | 0,13 ± 0,13 |
| Biodisponibilité (*µg*/*cm² de peau*) | 9,0 ±4,8 | 8,7 ± 4,0 |

Il a également été observé que le passage systémique de l'amitriptyline était inférieur à 0,1% de la dose administrée. Il en résulte que le passage systémique de l'amitriptyline est négligeable.

On remarque que le gel aqueux A selon l'invention présente un profil de pénétration de l'amitriptyline dans la peau satisfaisant et similaire au profil de pénétration de l'amitriptyline dans la peau obtenu avec la crème comparative B.

On note également que la biodisponibilité obtenue de la composition A et celle obtenue de la composition B sont similaires.

### Exemple 2 :

Une autre composition pharmaceutique sous forme de gel aqueux selon l'invention (composition A') a été préparée à partir des ingrédients indiqués dans le tableau ci-après dont les quantités sont exprimées en % en poids.

**[Tableau 3]**

| **COMPOSITION A' (Invention)** | **Quantité** |
|---|---|
| Chlorhydrate d'amitriptyline | 15 |
| Hydroxyéthylcellulose | 1 |
| Propylène glycol | 5 |
| Methyl paraben | 0,1 |
| Agent de pH | Qsp pH 5,5 ± 0,5 |
| Eau | Qsp 100 |

Il a été observé que le gel aqueux A' selon l'invention présente un profil de pénétration de l'amitriptyline dans la peau et une biodisponibilité en amitriptyline satisfaisants.

### Exemple 3 :

Etude dela stabilité d'une formulation C sous forme de gel aqueux (invention) et de la formulation B sous forme de crème (comparatif).

Le gel aqueux (composition C) suivant a été préparé à partir des ingrédients indiqués dans le tableau ci-après dont les quantités sont exprimées en % en poids.

**[Tableau 4]**

| **COMPOSITION C (Invention)** | **Quantité** |
|---|---|
| Chlorhydrate d'amitriptyline | 10 |
| Hydroxyéthylcellulose | 1 |
| Propylène glycol | 5 |
| PEG-7 Cocoate de glycéryle | 2 |
| Agent de pH (NaOH, solution 1N) | Qsp pH 5,5 ± 0,5 |
| Eau | Qsp 100 |

La composition B (crème) comprend 10% en poids de chlorhydrate d'amitriptyline et 90% en poids de crème Excipial Hydrocrème^{®}, commercialisée par la société Galderma, par rapport au poids total de la composition B.

Chacune des compositions B et C ont été placées dans une étuve à une température de 40°C.

La stabilité des compositions a ensuite été évaluée visuellement dans le temps (à T₀, au moment de l'entrée en étuve ; à T₂₄ₕ, 24 heures après l'entrée en étuve ; et à T₃ₘₒᵢₛ, 3 mois après l'entrée en étuve).

Les résultats sont regroupés dans le tableau ci-dessous.

**[Tableau 5]**

| | **Aspect** | | |
|---|---|---|---|
| **Compositions** | à T₀ | à T₂₄ₕ | à T₃ₘₒᵢₛ |
| Composition B (Comparatif) | Emulsion blanche opaque huile-dans-eau | Déphasage observé. La phase huileuse supérieure est blanche et opaque. La phase aqueuse inférieure est transparente | ND |
| Composition C (Invention) | Gel translucide sans couleur | Gel translucide | Gel translucide |

Aucune synérèse n'a été observé pour la composition C sous forme de gel aqueux selon l'invention après 3 mois à 40°C.

On remarque également un déphasage de la composition comparative B sous forme d'émulsion huile-dans-eau au bout de seulement 24 heures à 40°C.

De plus, une étude de stabilité complète a été effectué sur la composition C selon l'invention pendant 6 mois à 40°C.

Les résultats sont regroupés dans les tableaux 6 et 7 ci-après.

**[Tableau 6]**

| **TESTS** | **SPECIFI-CATIONS** | **RESULTATS** | | | |
|---|---|---|---|---|---|
| | | **T0** | **T1 mois** | **T3 mois** | **T6 mois** |
| Aspect visuel | Gel translucide sans couleur | Gel translucide sans couleur | Gel translucide sans couleur | Gel translucide sans couleur | Gel translucide sans couleur |
| pH | 4.5 à 6.0 | 5.5 | 5.6 | 5.5 | 5.3 |
| Viscosité | 450 à 1500 mPa.s | 1039 mPa.s | 883 mPa.s | 765 mPa.s | 756 mPa.s |
| Dosage de amitriptyline HCl | 95,0 mg/g à 105,0 mg/g | 102,7 mg/g | 99,9 mg/g | 99,6 mg/g | 100,6 mg/g |

**[Tableau 7]**

| **Produits de dégradation de l'amitriptyline HCl** | **SPECIFI-CATIONS** | **RESULTATS** | | | |
|---|---|---|---|---|---|
| | | **T0** | **T1 mois** | **T3 mois** | **T6 mois** |
| Cyclobenzaprine | ≤ 0,1% | < limite de détection | < limite de détection | < limite de détection | < limite de détection |
| Dibenzosuberone | ≤ 0,1% | Non détectée | Non détectée | Non détectée | Non détectée |
| Impuretés inconnues | Reportée si ≤ 0,1%, | Non détectée | Non détectée | Non détectée | Impureté inconnue 1 RRT 0,38 min < 0,1% ; |
| | Aucune < 0,2% | | | | Impureté inconnue 2 RRT 0,45 min < 0,1 % |
| Total Impuretés | ≤ 1 % | N/A | N/A | N/A | < 0,1 % |

Aucune synérèse n'a été observé pour la composition C sous forme de gel aqueux selon l'invention après 6 mois à 40°C.

On observe également aucune variation majeure sur chacun des tests réalisés.

On peut ainsi noter la bonne stabilité physico-chimique des compositions sous forme de gel aqueux selon l'invention.

## Revendications

1. Composition pharmaceutique sous forme de gel aqueux pour une application topique comprenant :
(i) au moins de l'amitriptyline et/ou l'un de ses sels pharmaceutiquement acceptable, dont la teneur totale en amitriptyline et/ou en l'un de ses sels pharmaceutiquement acceptable est comprise entre 10% et 30% en poids par rapport au poids total de la composition ;
(ii) de l'eau ;
(iii) au moins un polymère cellulosique ; et
(iv) au moins un polyol en C₂-C₈ ;
et le pH de la composition étant compris entre 4 et 7.

2. Composition selon la revendication précédente, **caractérisée en ce que** la teneur totale en amitriptyline et/ou en l'un de ses sels pharmaceutiquement acceptable est comprise entre 10 et 25% en poids, de préférence entre 10 et 20% en poids, plus préférentiellement encore entre 10 et 15% en poids, par rapport au poids total de la composition.

3. Composition selon l'une quelconque des revendications précédentes, **caractérisée en ce que** le ou les polymères cellulosiques ne comportent pas de chaîne grasse latérale en C₁₀-C₃₀ dans sa structure.

4. Composition selon l'une quelconque des revendications précédentes, **caractérisée en ce que** le ou les polymères cellulosiques présentent un poids moléculaire moyen compris entre 5 000 et 1 500 000, de préférence entre 50 000 et 800 000, plus préférentiellement entre 400 000 et 800 000.

5. Composition selon l'une quelconque des revendications précédentes, **caractérisée en ce que** le ou les polymères cellulosiques sont choisis parmi les éthers de cellulose ; de préférence parmi les éthers de cellulose non-ioniques ; plus préférentiellement parmi (a) les (C₁-C₄)alkylcelluloses telles que les méthylcelluloses et les éthylcelluloses, (b) les (poly)hydroxy(C₁-C₄)alkylcelluloses telles que les hydroxyméthylcelluloses, les hydroxyéthylcelluloses et les hydroxypropylcelluloses, (c) les celluloses mixtes (poly)hydroxy(C₁-C₄)alkyl-(C₁-C₄)alkylcelluloses telles que les hydroxypropyl-méthylcelluloses, les hydroxyéthyl-méthylcelluloses, les hydroxypropyl-éthylcelluloses, les hydroxyéthyl-éthylcelluloses et les hydroxybutyl-méthylcelluloses, et (d) leurs mélanges ; plus préférentiellement encore la composition comprend au moins une (poly)hydroxy(C₁-C₄)alkylcellulose telles que les hydroxyméthylcelluloses, les hydroxyéthylcelluloses et les hydroxypropylcelluloses ; et encore mieux la composition comprend au moins l'hydroxyéthylcellulose.

6. Composition selon l'une quelconque des revendications précédentes, **caractérisée en ce que** la teneur totale en polymère(s) cellulosique(s) est comprise entre 0,1 et 10% en poids, de préférence entre 0,5 et 5% en poids, plus préférentiellement encore entre 1 et 2,5% en poids, par rapport au poids total de la composition.

7. Composition selon l'une quelconque des revendications précédentes, **caractérisée en ce que** le ou les polyol(s) en C₂-C₈ sont choisis parmi les polyols en C₃-C₆, l'éthylène glycol, et leurs mélanges ; de préférence parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés ; plus préférentiellement la composition comprend au moins le propylène glycol.

8. Composition selon l'une quelconque des revendications précédentes, **caractérisée en ce que** la teneur totale en polyol(s) en C₂-C₈ est comprise entre 0,1 et 15% en poids, de préférence entre 0,5 et 10% en poids, plus préférentiellement encore entre 1 et 6% en poids, et encore mieux entre 3 et 6% en poids, par rapport au poids total de la composition.

9. Composition selon l'une quelconque des revendications précédentes, **caractérisée en ce que** la viscosité, à une température de 20°C et à pression atmosphérique, est comprise entre 400 et 2500 mPa.s ; de préférence entre 900 et 2000 mPa.s ; et plus préférentiellement entre 1000 et 1500 mPa.s.

10. Composition selon l'une quelconque des revendications précédentes, **caractérisée en ce qu'**elle est exempte d'agent séquestrant et/ou d'agent anti-oxydant.

11. Composition selon l'une quelconque des revendications précédentes, **caractérisée en ce que** le pH est compris entre 5 et 6.

12. Composition selon l'une quelconque des revendications précédentes, **caractérisée en ce que** la teneur en eau est supérieure ou égale à 65% en poids, de préférence comprise entre 65 et 90% en poids ; plus préférentiellement entre 70 et 90% en poids, plus préférentiellement encore entre 75 et 85% en poids, par rapport au poids total de la composition.

13. Composition selon l'une quelconque des revendications précédentes, **caractérisée en ce qu'**elle est exempte de corps gras.

14. Composition selon l'une quelconque des revendications précédentes pour son utilisation en tant que médicament.

15. Composition selon l'une quelconque des revendications précédentes pour son utilisation par voie topique dans le traitement des douleurs neuropathiques ; de préférence pour son utilisation par voie topique dans le traitement des douleurs neuropathiques périphériques post-chimiothérapie, des douleurs neuropathiques post-zostériennes, des douleurs neuropathiques diabétiques ; plus préférentiellement pour son utilisation par voie topique dans le traitement des douleurs neuropathiques périphériques post-chimiothérapie.

16. Composition selon l'une quelconque des revendications précédentes pour son utilisation dans le traitement de cancers comportant des séances de chimiothérapie, la composition étant administrée par voie topique entre les séances de chimiothérapie pour remédier ou prévenir des douleurs neuropathiques susceptibles d'être induites par la chimiothérapie.

17. Composition selon l'une quelconque des revendications 1 à 14 pour son utilisation par voie topique dans le traitement de l'érythromélalgie.

## Patentansprüche

1. Pharmazeutische Zusammensetzung in Form eines wässrigen Gels für die topische Anwendung, umfassend:
(i) mindestens Amitriptylin und/oder eines seiner pharmazeutisch akzeptablen Salze, dessen Gesamtgehalt an Amitriptylin und/oder einem seiner pharmazeutisch akzeptablen Salze zwischen 10 und 30 Gew.-%, bezogen auf das Gesamtgewicht der Zusammensetzung, liegt;
(ii) Wasser;
(iii) mindestens ein Cellulosepolymer; und
(iv) mindestens ein C₂-C₈-Polyol;
und wobei der pH-Wert der Zusammensetzung zwischen 4 und 7 liegt.

2. Zusammensetzung nach dem vorhergehenden Anspruch, **dadurch gekennzeichnet, dass** der Gesamtgehalt an Amitriptylin und/oder einem seiner pharmazeutisch akzeptablen Salze zwischen 10 und 25 Gew.-%, vorzugsweise zwischen 10 und 20 Gew.-%, noch bevorzugter zwischen 10 und 15 Gew.-%, bezogen auf das Gesamtgewicht der Zusammensetzung, liegt.

3. Zusammensetzung nach einem der vorhergehenden Ansprüche, **dadurch gekennzeichnet, dass** das oder die Cellulosepolymere in ihrer Struktur keine seitliche C₁₀-C₃₀-Fettkette aufweisen.

4. Zusammensetzung nach einem der vorhergehenden Ansprüche, **dadurch gekennzeichnet, dass** das oder die Cellulosepolymere ein durchschnittliches Molekulargewicht zwischen 5 000 und 1 500 000, vorzugsweise zwischen 50 000 und 800 000, noch bevorzugter zwischen 400 000 und 800 000, aufweisen.

5. Zusammensetzung nach einem der vorhergehenden Ansprüche, **dadurch gekennzeichnet, dass** das oder die Cellulosepolymere unter Celluloseethern ausgewählt sind; vorzugsweise unter nichtionischen Celluloseethern; noch bevorzugter unter (a) (C₁-C₄)Alkylcellulosen wie Methylcellulosen und Ethylcellulosen, (b) (Poly) hydroxy (C₁-C₄) alkylcellulosen wie Hydroxymethylcellulosen, Hydroxyethylcellulosen und Hydroxypropylcellulosen, (c) gemischten (Poly)hydroxy(C₁-C₄)alkyl-(C₁-C₄)alkylcellulosen wie Hydroxypropylmethylcellulosen, Hydroxyethylmethylcellulosen, Hydroxypropylethylcellulosen, Hydroxyethylethylcellulosen und Hydroxybutylmethylcellulosen, und (d) deren Mischungen; noch bevorzugter umfasst die Zusammensetzung mindestens eine (Poly) hydroxy (C₁-C₄) alkylcellulose wie Hydroxymethylcellulosen, Hydroxyethylcellulosen und Hydroxypropylcellulosen; und noch besser umfasst die Zusammensetzung mindestens Hydroxyethylcellulose.

6. Zusammensetzung nach einem der vorhergehenden Ansprüche, **dadurch gekennzeichnet, dass** der Gesamtgehalt an Cellulosepolymer(en) zwischen 0,1 und 10 Gew.-%, vorzugsweise zwischen 0,5 und 5 Gew.-%, noch bevorzugter zwischen 1 und 2,5 Gew.-%, bezogen auf das Gesamtgewicht der Zusammensetzung, liegt.

7. Zusammensetzung nach einem der vorhergehenden Ansprüche, **dadurch gekennzeichnet, dass** das oder die C₂-C₈-Polyole aus C₃-C₆-Polyolen, Ethylenglykol und deren Gemischen; vorzugsweise aus Propylenglykol, 1,3-Propandiol, 1,3-Butylenglykol, Pentan-1,2-diol, Dipropylenglykol, Hexylenglykol, Pentylenglykol, Glycerin, Ethylenglykol und einer Mischung dieser Verbindungen ausgewählt sind; wobei die Zusammensetzung noch bevorzugter mindestens Propylenglykol umfasst.

8. Zusammensetzung nach einem der vorhergehenden Ansprüche, **dadurch gekennzeichnet, dass** der Gesamtgehalt an C₂-C₈-Polyol(en) zwischen 0,1 und 15 Gew.-%, vorzugsweise zwischen 0,5 und 10 Gew.-%, noch bevorzugter zwischen 1 und 6 Gew.-% und noch besser zwischen 3 und 6 Gew.-%, bezogen auf das Gesamtgewicht der Zusammensetzung, liegt.

9. Zusammensetzung nach einem der vorhergehenden Ansprüche, **dadurch gekennzeichnet, dass** die Viskosität bei einer Temperatur von 20 °C und bei Atmosphärendruck zwischen 400 und 2500 mPa.s, vorzugsweise zwischen 900 und 2000 mPa.s und noch bevorzugter zwischen 1000 und 1500 mPa.s, liegt.

10. Zusammensetzung nach einem der vorhergehenden Ansprüche, **dadurch gekennzeichnet, dass** sie frei von Sequestriermittel und/oder Antioxidationsmittel ist.

11. Zusammensetzung nach einem der vorhergehenden Ansprüche, **dadurch gekennzeichnet, dass** der pH-Wert zwischen 5 und 6 liegt.

12. Zusammensetzung nach einem der vorhergehenden Ansprüche, **dadurch gekennzeichnet, dass** der Wassergehalt größer oder gleich 65 Gew.-% ist und vorzugsweise zwischen 65 und 90 Gew.-%; noch bevorzugter zwischen 70 und 90 Gew.-%, noch bevorzugter zwischen 75 und 85 Gew.-%, bezogen auf das Gesamtgewicht der Zusammensetzung, liegt.

13. Zusammensetzung nach einem der vorhergehenden Ansprüche, **dadurch gekennzeichnet, dass** sie frei von Fettkörpern ist.

14. Zusammensetzung nach einem der vorhergehenden Ansprüche zur Anwendung als Arzneimittel.

15. Zusammensetzung nach einem der vorhergehenden Ansprüche für ihre topische Anwendung bei der Behandlung neuropathischer Schmerzen; vorzugsweise für ihre topische Anwendung bei der Behandlung peripherer neuropathischer Schmerzen nach Chemotherapie, postzosterischer neuropathischer Schmerzen, diabetischer neuropathischer Schmerzen; noch bevorzugter für ihre topische Anwendung bei der Behandlung von peripheren neuropathischen Schmerzen nach der Chemotherapie.

16. Zusammensetzung nach einem der vorhergehenden Ansprüche zur Anwendung bei der Behandlung von Krebs mit Chemotherapiesitzungen, wobei die Zusammensetzung topisch zwischen Chemotherapiesitzungen verabreicht wird, um neuropathische Schmerzen zu lindern oder zu verhindern, die durch die Chemotherapie verursacht werden können.

17. Zusammensetzung nach einem der Ansprüche 1 bis 14 zur topischen Anwendung bei der Behandlung von Erythromelalgie.

## Claims

1. Pharmaceutical composition in the form of an aqueous gel for topical application comprising:
(i) at least amitriptyline and/or one of its pharmaceutically acceptable salts, wherein the total content of amitriptyline and/or of one of its pharmaceutically acceptable salts ranges from 10% to 30% by weight relative to the total weight of the composition;
(ii) water;
(iii) at least one cellulose polymer; and
(iv) at least one C₂-C₈ polyol;
and the pH of the composition ranging from 4 to 7.

2. Composition according to the preceding claim, **characterised in that** the total content of amitriptyline and/or of one of its pharmaceutically acceptable salts ranges from 10 to 25% by weight, preferably from 10 to 20% by weight, more preferably still from 10 to 15% by weight, relative to the total weight of the composition.

3. Composition according to any one of the preceding claims, **characterised in that** the cellulose polymer(s) do not include a C₁₀-C₃₀ lateral fatty chain in its structure.

4. Composition according to any one of the preceding claims, **characterised in that** the cellulose polymer(s) have an average molecular weight between 5 000 and 1 500 000, preferably between 50 000 and 800 000, more preferentially between 400 000 and 800 000.

5. Composition according to any one of the preceding claims, **characterised in that** the cellulose polymer(s) are chosen from cellulose ethers; preferably from nonionic cellulose ethers; more preferably from (a) (C₁-C₄) alkylcelluloses such as methylcelluloses and ethylcelluloses, (b) (poly) hydroxy (C₁-C₄) alkylcelluloses such as hydroxymethylcelluloses, hydroxyethylcelluloses and hydroxypropylcelluloses, (c) (poly) hydroxy (C₁-C₄) alkyl-(C₁-C₄)alkylcelluloses mixed celluloses such as hydroxypropyl-methylcelluloses, hydroxyethyl-methylcelluloses, hydroxypropyl-ethylcelluloses, hydroxyethyl-ethylcelluloses, and hydroxybutyl-methylcelluloses, and (d) mixtures thereof; more preferably still, the composition comprises at least one (poly) hydroxy (C₁-C₄) alkylcellulose such as hydroxymethylcelluloses, hydroxyethylcelluloses, and hydroxypropylcelluloses; and even better, the composition comprises at least hydroxyethylcellulose.

6. Composition according to any of the preceding claims, **characterised in that** the total content of cellulose polymer(s) ranges from 0.1 to 10% by weight, preferably from 0.5 to 5% by weight, more preferably still from 1 to 2.5% by weight, relative to the total weight of the composition.

7. Composition according to any one of the preceding claims, **characterised in that** the C₂-C₈ polyol(s) are chosen from C₃-C₆ polyols, ethylene glycol, and mixtures thereof; preferably from propylene glycol, 1,3-propanediol, 1,3-butylene glycol, pentane-1,2-diol, dipropylene glycol, hexylene glycol, pentylene glycol, glycerol, ethylene glycol, and a mixture of these compounds; more preferably the composition comprises at least propylene glycol.

8. Composition according to any one of the preceding claims, **characterised in that** the total content of C₂-C₈ polyol(s) ranges from 0.1 to 15% by weight, preferably from 0.5 to 10% by weight, more preferably still from 1 to 6% by weight, and even better from 3 to 6% by weight, relative to the total weight of the composition.

9. Composition according to any one of the preceding claims, **characterised in that** the viscosity, at a temperature of 20°C and at atmospheric pressure, ranges from 400 to 2 500 mPa.s; preferably from 900 to 2 000 mPa.s; and more preferentially from 1 000 to 1 500 mPa.s.

10. Composition according to any one of the preceding claims, **characterised in that** it is free from sequestering agent and/or antioxidant agent.

11. Composition according to any one of the preceding claims, **characterised in that** the pH ranges from 5 to 6.

12. Composition according to any of the preceding claims, **characterised in that** the content of water is greater than or equal to 65% by weight, preferably ranges from 65 to 90% by weight, more preferentially from 70 to 90% by weight, more preferentially still from 75 to 85% by weight, relative to the total weight of the composition.

13. Composition according to any one of the preceding claims, **characterised in that** it is free from fatty substance.

14. Composition according to any one of the preceding claims for use as a medicament.

15. Composition according to any one of the preceding claims for topical use in the treatment of neuropathic pain; preferably for topical use in the treatment of post-chemotherapy peripheral neuropathic pain, post-zoster neuropathic pain, diabetic neuropathic pain; more preferably for topical use in the treatment of post-chemotherapy peripheral neuropathic pain.

16. Composition according to any one of the preceding claims for use in the treatment of cancers including chemotherapy sessions, the composition being administered topically between the chemotherapy sessions to remedy or prevent neuropathic pain likely to be caused by chemotherapy.

17. Composition according to any one of claims 1 to 14 for topical use in the treatment of erythromelalgia.
